# EUROPEAN PATENT APPLICATION

(11) **EP 2 113 229 A1**
(43) Date of publication of application: **04.11.2009**
(21) Application number: 08703599.4
(22) Date of filing: 22.01.2008
(51) Int. Cl.: A61F 2/84, A61F 2/04, A61F 2/06

(54) **STENT AND TREATMENT DEVICE FOR TUBULAR ORGAN**

(30) Priority: 20.02.2007 JP 2007040035
(71) Applicant: National University Corporation University of Fukui, Fukui-shi, Fukui 910-8507 (JP); Sapporo Medical University, Hokkaido 060-0061 (JP)
(72) Inventor: SAKAI, Toyohiko, Fukui-shi Fukui 910-8507 (JP); HYODOH, Hideki, Sapporo-shi Hokkaido 060-8556 (JP)
(74) Representative: Whalley, Kevin
(86) International application number: PCT/JP2008/050749
(87) International publication number: WO 2008/102589

(57) **Abstract**

The present invention provides a stent and tubular organ treatment device that can flexibly deform according to a curved portion of a tubular organ, contracts less in the longitudinal direction during insertion, and is easy to handle. A stent (1) is formed into a tubular form by arranging five tubular unit bodies in an axial direction and connecting them by a connecting filament (F). Each tubular unit is formed into a closed loop by cutting a metal wire to a predetermined length, bending it in a zigzag manner in a peripheral direction, and securely connecting both ends thereof. A tubular unit body (W) having two bent portions projected in the axial direction is connected to and rockably retained by a tubular unit body (U1). Similarly, a tubular unit body (V) is rockably retained by the tubular unit body (W), whereby the entire stent can easily deform into a curved shape. Shape-retaining members (X1) and (X2) composed of a shape memory alloy material are attached in the axial direction to the tubular unit bodies to maintain them in a curved state.

## Description

### Technical Field

The present invention relates to a stent inserted into a tubular organ of a human body, such as a blood vessel, a ureter, a bile duct, or a trachea, to keep a lumen of the tubular organ open, and also relates to a tubular-organ treatment device using the same.

### Background Art

In recent years, when a lumen of a tubular organ of a human body, such as a blood vessel, is blocked, in order to expand and reopen the lumen or reinforce the weakened lumen, a treatment in which a catheter is passed through the tubular organ to insert and position a stent in a lesion is performed. For example, as an approach to treat aortic aneurysm, a treatment method in which a treatment device, i.e., a stent having the outer peripheral surface covered with a graft, is inserted into a lesion and the stent is expanded in a blood vessel to attach the graft to the inner peripheral surface of the blood vessel has been studied.

In a treatment method employing such a stent, although insertion and positioning can be easily performed at a straight tubular portion of a tubular organ of a human body, insertion and positioning of the stent is difficult at a curved portion. Thus, various improvements have been made.

For example, Patent Document 1 discloses a stent formed of an elastic wire deformed in a zigzag manner and spirally wound, anchoring portions of bent portions being tied with thread, so as to be formed into a curved cylinder shape. Patent Document 2 discloses a stent formed of a first wire and second wire composed of a super-elastic shape memory alloy material, the first and second wires being bent in a zigzag manner in a longitudinal direction and entangled with each other at bent portions so as to be formed into a mesh structure. Patent Document 3 discloses a stent formed of a metal wire woven into a tubular shape, the stent having a woven structure in which U-shaped bent portions of the metal wire are entangled with cross portions. Patent Document 4 discloses that Z - stents are attached to the inner peripheral surfaces on both sides of a tube made of a graft material, a Z-stent is disposed on the outer peripheral surface of the middle portion, and both ends of an elastic material are attached to the tube so that the length is contracted.
Patent Document 1: Japanese Unexamined Patent Application Publication No. 9-164209
Patent Document 2: Japanese Unexamined Patent Application Publication No. 2004-344634
Patent Document 3: Japanese Unexamined Patent Application Publication No. 2004-49806
Patent Document 4: Japanese Translation of PCT International Application, Publication No. 2005-506874

### Disclosure of Invention

### Problems to be Solved by the Invention

In Patent Document 1, although the stent can be formed into a curved shape, flexible deformation according to the shape of a tubular organ is difficult. Further, because the curve of an installation site needs to be measured in advance, it is difficult to cope with an urgent case.

In Patent Documents 2 and 3, the bent portions of the metal wires are entangled. When the stent is curved, the entangled portions are shifted, allowing the stent to easily deform into a curved shape. However, because a long metal wire extending over the entire length of the stent is used in a bent state, the metal wire accommodated in a catheter is in an elongated state. When such an elongated metal wire is released in a blood vessel, the entire stent is contracted in the longitudinal direction. This makes handling difficult.

In Patent Document 4, a plurality of Z-stents are arranged in the longitudinal direction. However, because the adjacent Z-stents are not connected, they are susceptible to deformation in the longitudinal direction. Thus, handling during insertion by a catheter is difficult.

Therefore, an object of the present invention is to provide a stent which can flexibly deform with respect to a curved portion of a tubular organ, contracts less in the longitudinal direction during insertion, and is easy to handle, and to provide a tubular-organ treatment device using the same.

### Means for Solving the Problems

A stent according to the present invention is a stent formed into a tubular form, including a plurality of tubular unit bodies arranged in an axial direction, the plurality of tubular unit bodies each being composed of a wire formed into a closed loop and bent in a zigzag manner in a peripheral direction to form a plurality of bent portions spaced apart from one another on both sides, the bent portions each having an anchoring portion, the anchoring portions of the adjacent tubular unit bodies, facing each other, being connected by a connecting filament. At least some of the tubular unit bodies are connected to the adjacent tubular unit bodies only at the anchoring portions of some of the bent portions of a bent portion group on at least one side so as to be rockably retained. In addition, the bent portions connected to retain rockably are projected in the axial direction compared to the other bent portions. In addition, there is provided an elastic shape-retaining member that is attached to the plurality of tubular unit bodies including the rockably retained tubular unit bodies and allows the tubular unit bodies to rock to maintain them in a curved state. In addition, the shape-retaining member is formed of a curved elastic wire having both ends fixed to the tubular unit bodies, the shape-retaining member being retained by the connecting filament stretched in the peripheral direction so as to conform to the external shape of the stent in the axial direction. In addition, a pair of the shape-retaining members are fixed at one end to the tubular unit body so as to be spaced apart from each other, crossed over each other, and fixed at the other end to the tubular unit body so as to be spaced apart from each other. In addition, the shape-retaining member is composed of a shape memory alloy material.

A tubular organ treatment device of the present invention includes the above-described stent and a tubular cover that covers an inner peripheral surface and/or outer peripheral surface of the stent.

### Advantages

In the present invention, because of the above-described structure, since at least some of the tubular unit bodies are connected to and rockably retained by the tubular unit bodies adjacent thereto only at the anchoring portions of some of the bent portions of at least one of the bent portion groups, by inserting and positioning the rockably retained tubular unit bodies into a curved portion of a tubular organ, the tubular unit bodies rock so as to conform to the curved portion and can easily deform into a curved shape. Because the tubular unit bodies rock and deform, even when the curve of the tubular organ differs from person to person, it is possible to flexibly cope with it and easily deform into a curved shape. It also becomes easy to prepare the stent in advance and cope with an urgent treatment.

Furthermore, because the plurality of tubular unit bodies, each composed of a wire formed into a closed loop and bent in a zigzag manner in the peripheral direction to form a plurality of bent portions on both sides, the bent portions each having an anchoring portion, are arranged in the axial direction, the tubular unit bodies can expand and contract from the center in the radial direction, and, when they deform from a contracted state to an expanded state, the length in the axial direction does not significantly change. This enables easy handling during insertion by a catheter. In addition, because each tubular unit body is connected to the adjacent tubular unit body by the connecting filament, they have sufficient durability against deformation in the axial direction and are maintained in a stable shape when inserted and positioned in a tubular organ.

In addition, to retain rockably, by forming the connected bent portions so as to be projected in the axial direction compared to the other bent portions, the rocking range of the tubular unit bodies can be increased. Thus, it is possible to sufficiently cope with a sharp curve.

Furthermore, by providing the elastic shape-retaining member that is attached to the plurality of tubular unit bodies including the rockably retained tubular unit bodies and allows the tubular unit bodies to rock to maintain them in a curved state, the curve of the stent can be stably maintained. Because the shape-retaining member has elasticity, during insertion of the stent, deformation into a straight tubular form can be easily performed. Thus, convenience of handling improves.

Furthermore, by forming the shape-retaining member of a curved elastic wire, fixing both ends thereof to the tubular unit bodies, and retaining the shape-retaining member by the connecting filament stretched in the peripheral direction so as to conform to the external shape of the stent in the axial direction, both ends of the shape-retaining member are fixed to the tubular unit bodies so as not to be displaced easily, and by holding the middle portion, without fixing it, by the connecting filament stretched in the peripheral direction so as to conform to the external shape of the stent, when the entire stent is deformed into a straight tubular form, the shape-retaining member is sifted and easily deformed into a straight shape.

Furthermore, by fixing a pair of the shape-retaining members, spaced apart from each other, at one end to the tubular unit body, crossing them over each other, and fixing them, at the other end, to the tubular unit body so as to be spaced apart from each other, resistance to deformation, such as twist of the stent, is increased, the curve can be more stably maintained, and the strength of the entire stent can be improved.

Furthermore, by using a shape memory alloy material as the shape-retaining member, when positioned in a tubular organ, a desirable curve can be formed.

### Best Mode for Carrying Out the Invention

Embodiments of the present invention will be described in detail below. Although the embodiments described below include various technical limitations since they are preferable examples for embodying the present invention, the present invention is not limited to such embodiments unless the following description specifies that the present invention is limited.

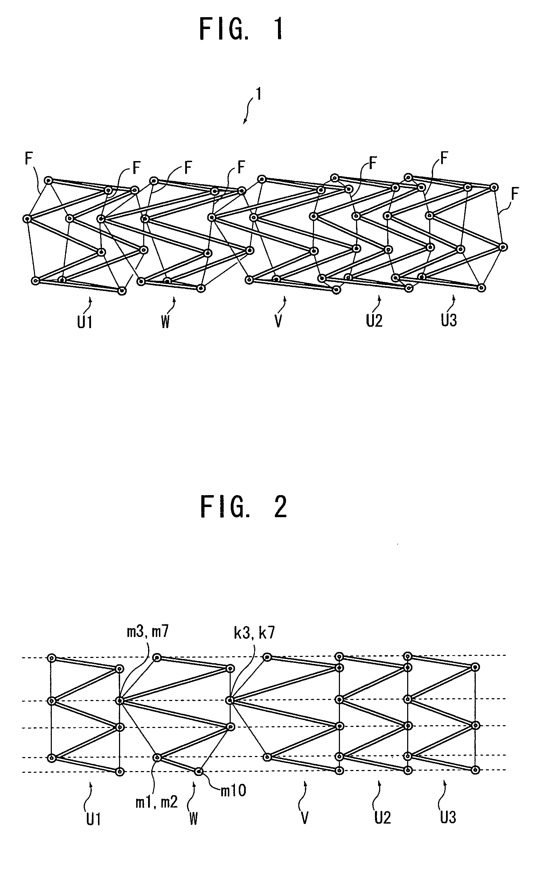

FIGS. 1 and 2 are a perspective view and side view according to an embodiment of the present invention. A stent 1 is formed of five tubular unit bodies U1, W, V, U2, and U3 arranged in the axial direction. The adjacent tubular unit bodies are connected to one another into a tubular form with a connecting filament F.

Each tubular unit is formed into a closed loop by cutting a metal wire to a predetermined length, bending it in a zigzag manner in the peripheral direction, and securely connecting both ends.

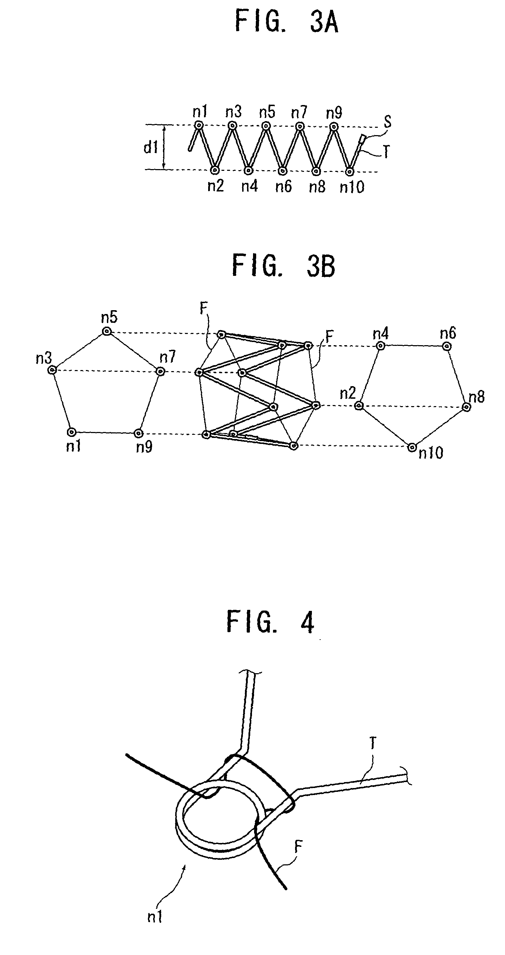

The tubular unit bodies U1 to U3 are used in a straight tubular portion. FIG. 3 shows an unfolded view (FIG. 3A) and a perspective view (FIG. 3B) thereof. A metal wire T is cut to a predetermined length and bent in a zigzag manner to have a width d1 to form bent portions. In this example, ten bent portions n₁ to n₁₀ are formed. Both ends of the wire T are securely connected at a connection portion S. At the connection portion S, both ends of the wire T may be secured using solder or the like. Other method for securely connecting metal wires may also be used.

At each bent portion, an anchoring portion, to which the connecting filament F is anchored, is formed. For example, as shown in FIG. 4, by circularly winding the bent portion twice into a torsion spring form to provide elasticity, and by entangling the connecting filament F in a manner that the connecting filament F passed through the circular portion from the inside to the outside is wrapped around the wire T and again passed through the circular portion from the inside to the outside, the connecting filament F is anchored to the circular portion, serving as the anchoring portion, without being disengaged. The anchoring portion may be formed into another shape. As long as the connecting filament F is not disengaged, any shape may be used.

When the wire T, having the bent portions n₁ to n₁₀, is connected at the connection portion S into a closed loop, each tubular unit body has the bent portions arranged at the vertices of a pentagon on both sides in the axial direction. A bent portion group, in which five bent portions, n₂, n₄, n₆, n₈ and n₁₀, are arranged, is defined on one side, and a bent portion group, in which five bent portions, n₁, n₃, n₅, n₇ and n₉, are arranged, is defined on the other side. The bent portions on both sides are arranged in a plane perpendicular to the axial direction.

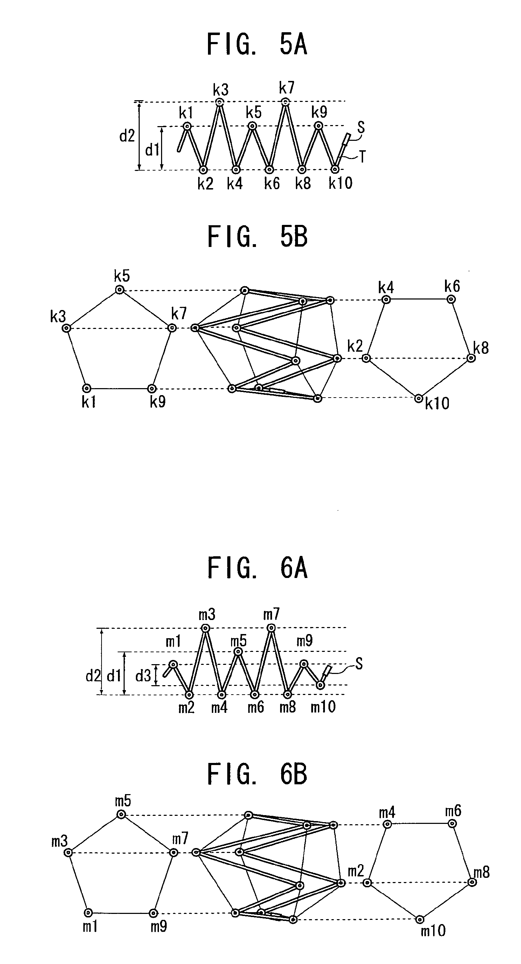

The tubular unit bodies V and W are used in a curved portion. FIG. 5 is an unfolded view (FIG. 5A) and perspective view (FIG. 5B) of the tubular unit body V. FIG. 6 is an unfolded view (FIG. 6A) and perspective view (FIG. 6B) of the tubular unit body V.

The tubular unit body V is formed of the metal wire T cut to a predetermined length and bent in a zigzag manner to have a width d1 or d2, and has ten bent portions k₁ to k₁₀. The width d2 is set to have a greater width than d1, and the metal wire T is bent to have a greater length at the bent portions k₃ and k₇, that is, bent such that the width is d2. Similarly to the tubular unit bodies U1 to U3, an anchoring portion, to which the connecting filament F is anchored, is formed at each bent portion. Similarly to the tubular unit bodies U1 to U3, the tubular unit body V is formed of the wire T, both ends thereof being securely connected at the connection portion S to be formed into a closed loop, and has, on both sides in the axial direction, a bent portion group, in which five bent portions, k₂, k₄, k_{6,} k₈ and k₁₀, are arranged at the vertices of a pentagon, on one side, and a bent portion group, in which five bent portions, k₁, k₃, k₅, k₇ and k₉, are arranged at the vertices of a pentagon, on the other side. Although the bent portions are arranged in a plane perpendicular to the axial direction on one side, the bent portions k₃ and k₇ are projected in the axial direction compared to the other bent portions on the other side.

The tubular unit body W is formed of the metal wire T cut to a predetermined length and bent in a zigzag manner to have a width d1, d2, or d3, and has ten bent portions m₁ to m₁₀. The width d2 is set to have a greater width than d1, and the width d3 is set to have a smaller width than d1. The metal wire T is bent to have a greater length at the bent portions m₃ and m₇, that is, bent such that the width is d2, and is bent to have a smaller length at the bent portions m₁, m₉ and m₁₀, that is, bent such that the width is d3. Similarly to the tubular unit bodies U1 to U3, an anchoring portion, to which the connecting filament F is anchored, is formed at each bent portion. Similarly to the tubular unit bodies U1 to U3, the tubular unit body W has the wire T, both ends thereof being securely connected at the connection portion S to be formed into a closed loop, and has, on both sides in the axial direction, a bent portion group, in which five bent portions, m₂ , m₄, m₆, m₈ and m₁₀, are arranged at the vertices of a pentagon, on one side, and a bent portion group, in which five bent portions, m₁, m₃, m₅, m₇ and m₉, are arranged at the vertices of a pentagon, on the other side. The bent portions m₂, m₄, m₆ and m₈ are arranged in a plane perpendicular to the axial direction and the bent portion m₁₀ is provided at a position inward of the plane on one side. On the other side, with respect to the plane perpendicular to the axial direction extending through the bent portions m₅, the bent portions m₃ and m₇ are provided so as to be projected in the axial direction, and the bent portions m₁ and m₉ are provided at positions inward of the plane.

The connecting filament F is stretched in the peripheral direction while being anchored to the bent portions on both sides of the tubular unit bodies and is connected at both ends into a closed loop. The tubular unit bodies can be deformed so as to contract toward the center by bending the bent portions, and are automatically returned from a contracted state to an expanded state by elasticity exerted by the torsion springs of the bent portions. Therefore, the tubular unit bodies can be maintained in an expanded state having a predetermined size by adjusting the length of the connecting filament F anchored to both sides thereof. Accordingly, it becomes possible to preliminarily determine the size to which the tubular units are expanded when the stent is inserted into a tubular organ by a catheter.

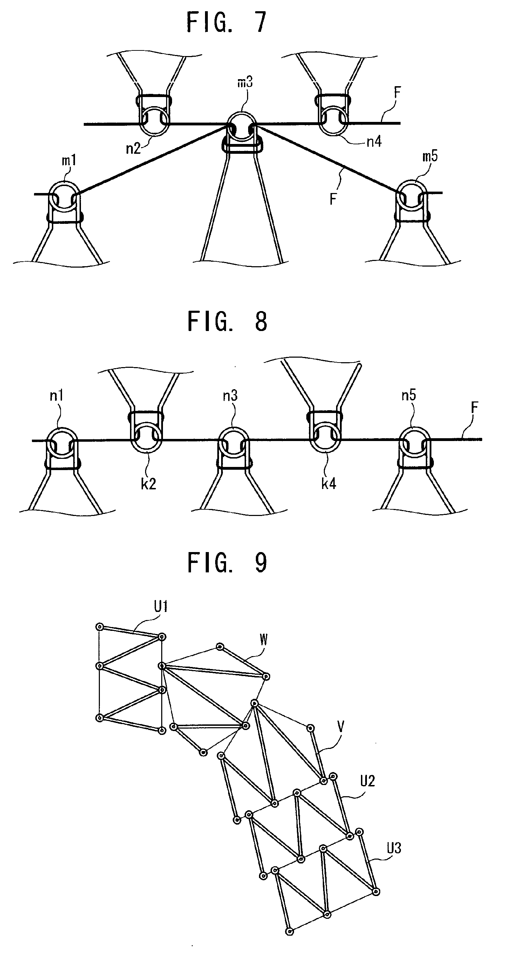

In this embodiment, the connecting filament F is stretched in the peripheral direction over the bent portion groups on both sides of the tubular unit bodies U1 and W. The bent portions m₃ and m₇, projecting in the axial direction of the tubular unit body W, are further connected to the connecting filament F anchored to the tubular unit body U1. As shown in FIG. 7, the bent portion m₃ of the tubular unit body W is connected by being entangled with the connecting filament F between the bent portions n₂ and n₄ of the tubular unit body U1, and similarly, the bent portions m₇ is connected by being entangled with the connecting filament F between bent portions n₆ and n₈. Thus, the tubular unit body W is retained rockably by the tubular unit body U1, using the bent portions m₃ and m₇ as the fulcrum. Because the bent portions m₃ and m₇ of the tubular unit body W are projected and the bent portions m₁ and m₉ are provided at inward positions, when the tubular unit body W rocks, a wide rocking range, to the extent that the bent portions m₁ and m₉ come into contact with the tubular unit body U1, can be provided. Thus, a sharp curve can be formed.

In the tubular unit body V, the connecting filament F is stretched in the peripheral direction over the bent portion group on the tubular unit body W side. The bent portions k₃ and k₇, projecting in the axial direction of the tubular unit body V, are connected to the connecting filament F anchored to the tubular unit body W. The bent portions k₃ of the tubular unit body V is connected by being entangled with the connecting filament F between the bent portions m₂ and m₄ of the tubular unit body W, and the bent portions k₇ is connected by being entangled with the connecting filament F between the bent portions m₆ and m₈. Thus, the tubular unit body V is retained rockably by the tubular unit body W, using the bent portions k₃ and k₇ as the fulcrum. Because the bent portions k₃ and k₇ of the tubular unit body V are projected and the bent portions m₁₀ of the tubular unit body W is provided at an inward position, when the tubular unit body V rocks, a wide rocking range, to the extent that the bent portions m₁ and m₉ come into contact with the tubular unit body W, can be provided. Thus, a sharp curve can be formed.

As shown in FIG. 8, the tubular unit bodies V and U2 are connected such that one connecting filament F is alternately anchored to the bent portions of the bent groups facing each other. The connecting filament F is stretched in the peripheral direction and connected at both ends into a closed loop. Therefore, the tubular unit bodies V and U2 are integrally connected into a straight tubular form without rocking. Similarly, the tubular unit bodies U2 and U3 are connected such that one connecting filament F is alternately anchored to the bent portions of the bent groups facing each other, and are integrally connected into a straight tubular form without rocking.

FIG. 9 is a side view showing the stent 1 in a curved state. Because the tubular unit bodies W and V are retained rockably, a desired curve can be easily formed by rocking them according to the curved shape. Because the tubular unit bodies can expand and contract from the center in the radial direction, and the length in the axial direction does not significantly change from a contracted state to an expanded state, the length of the entire stent 1 in the axial direction does not significantly change, and insertion and positioning in a curved tubular organ can be easily performed.

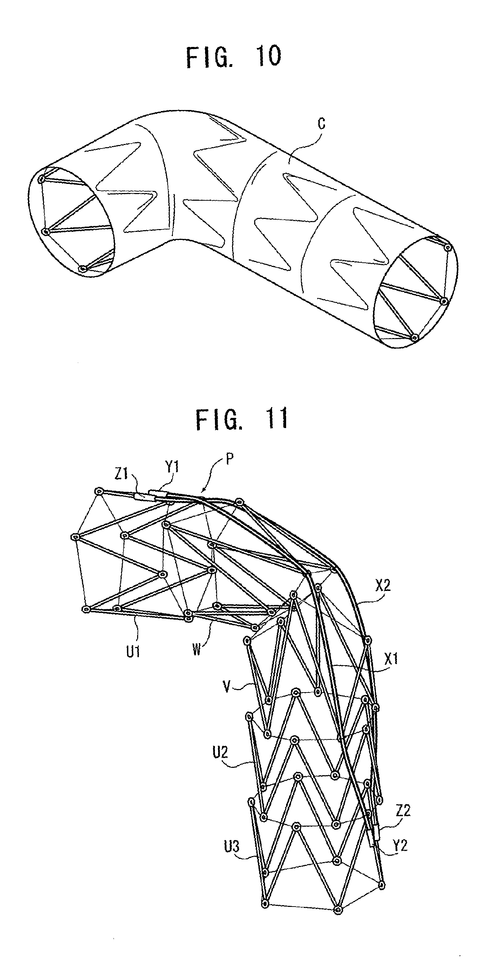

FIG. 10 shows a state in which the outer peripheral surface of the stent 1 is covered with a tubular cover. For example, by covering with a tubular cover for an artificial blood vessel, the use as a treatment device for a blood vessel is possible. The tubular cover may alternatively be provided so as to be fitted to the inner peripheral surface of the stent 1.

The wire used for the tubular unit bodies is preferably composed of an elastic material that is harmless to the human body and has excellent workability, such as stainless or an alloy of titanium and nickel. Alternatively, a resin material processed in a zigzag manner and formed into a closed loop may be used.

As the connecting filament F, a thread composed of a synthetic fiber, such as polypropylene, may be used.

Although the above-described tubular unit bodies are those having the bent portions arranged at the vertices of a pentagon on both sides, tubular unit bodies having the bent portions arranged at the vertices of another polygon may also be used. For example, if the number of vertices increases, like an octagon, the shape of the tubular unit bodies becomes cylindrical. This increases the entire strength and can further equalize the strength of the respective portions. In the tubular unit bodies constituting the curved portion, the position and number of the bent portions projecting in the axial direction may be appropriately determined according to the curved shape of the stent, and the length of projection can be appropriately determined according to the curvature of the curve. In addition, the width and size of the tubular unit bodies in the axial direction may be individually and appropriately determined, and they can be designed according to the shape of the tubular organ in which the tubular unit bodies are positioned.

By attaching an elastic shape-retaining member for retaining the curve to the above-described stent 1, the curved shape as shown in FIG. 9 can be stably maintained.

FIG. 11 is a perspective view according to an embodiment in which elastic wires formed into a curved shape are used as the shape-retaining members. Shape-retaining members X1 and X2 are formed of wires composed of a shape memory alloy material, such as nitinol, cut to a predetermined length and preliminarily formed into a curved shape, and are attached to the arranged tubular unit bodies from outside and positioned in the axial direction. The shape-retaining member X1 has one end securely connected to the wire of the tubular unit body U1 at a connection portion Y1 and has the other end securely connected to the wire of the tubular unit body U3 at a connection portion Y2. Similarly, the shape-retaining member X2 has one end securely connected to the wire of the tubular unit body U1 at a connection portion Z1 and has the other end securely connected to the wire of the tubular unit body U3 at a connection portion Z2. Accordingly, the tubular unit bodies W and V rock so as to conform to the curved shape of the shape-retaining members, and thus, the entire stent 1 is maintained in a curved state.

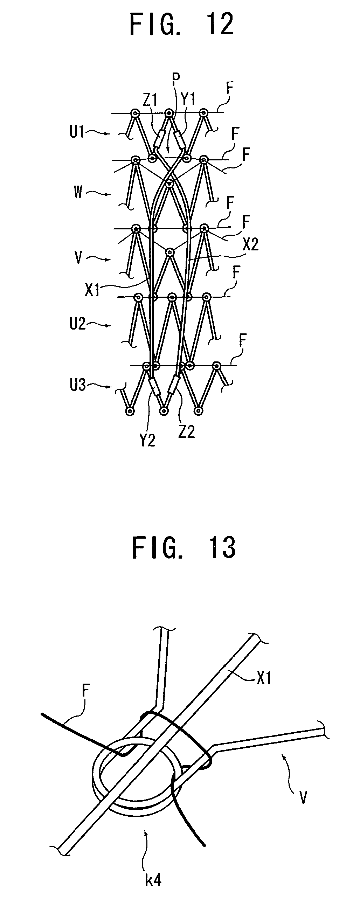

FIG. 12 is a plan view showing a mounting state of the shape-retaining members X1 and X2. The shape-retaining members X1 and X2 are securely connected at one end to the tubular unit body U1 so as to be a predetermined distance apart from each other, crossed over each other at a position P and arranged parallel with each other in the axial direction so as to be a predetermined distance apart from each other, and securely connected at the other end to the tubular unit body U3. The shape-retaining members X1 and X2, when crossing the connecting filament F, extend inside the connecting filament F stretched in the peripheral direction, and, at the bent portions, are each tightly held between the bent portion and the connecting filament F anchored to the anchoring portion, as shown in FIG. 13. Thus, the shape-retaining members are provided along the external shape of the stent 1 and retained in a stable state without being displaced in the peripheral direction.

Because the shape-retaining members can deform elastically, the stent can easily deform into a straight tubular form. Thus, easy handling during mounting of the stent on a catheter becomes possible.

As has been described, by attaching the shape-retaining members, the stent can be maintained in a predetermined curved state, and the strength of the stent with respect to torsion and the strength with respect to deformation in the axial direction can be increased. In particular, although the strength is low at the curved portion because the tubular unit bodies are rockably connected, by providing two shape-retaining members in a crossed manner, the strength of the curved portion can be increased.

As a shape-retaining member, a single elastic wire may be used. Besides wires, a resin material, such as silicon rubber, may be used and attached to the inside of the curved shape to maintain the shape. Alternatively, the shape-retaining member may be attached to the inside of the stent to keep the shape.

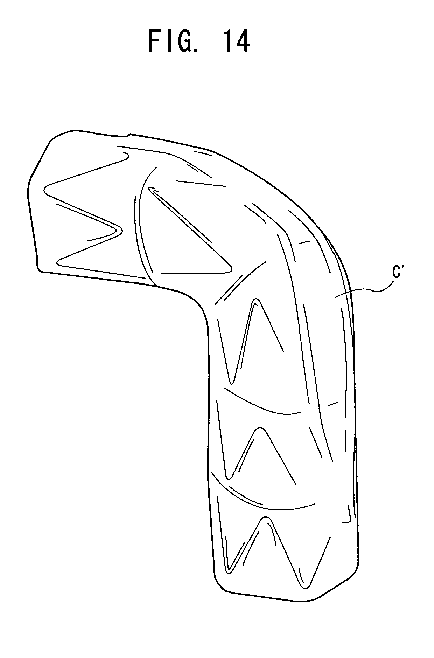

FIG. 14 is a perspective view showing a state in which the outer peripheral surface of the stent to which the shape-retaining members are attached is covered with a tubular cover C. Similarly to the case of FIG. 10, the tubular cover may be attached so as to be fitted to the inner peripheral surface of the stent.

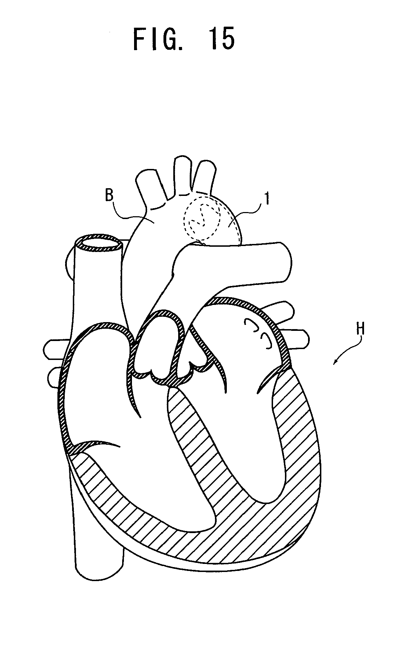

FIG. 15 is an explanatory diagram showing a state in which the stent 1 is inserted and positioned in an aorta B that carries blood from a heart H. The aorta B extends in a sharply curved manner from the top portion of the heart. Because the curved portion is subjected to the stream of the blood from the heart and tends to form an aneurysm at the top portion, the portion requires treatment for the aneurysm. In this case, by inserting and positioning the stent as shown in FIG. 11, effective treatment can be conducted. That is, by attaching the shape-retaining members formed into a curved shape according to the curve of the aorta to the stent, a curved stent can be easily formed. When positioned in the aorta, the shape-retaining members are bent to conform to the curved portion of the aorta. Thus, the shape can be adjusted according to the curve of each person's aorta, and it is possible to cope with an urgent treatment. In addition, because the shape-retaining members are positioned at the top portion of the inner surface of the aorta after being inserted and positioned, deformation due to the impact force of the stream of the blood does not occur and sufficient strength is provided.

### Brief Description of Drawings

[FIG. 1] FIG. 1 is a perspective view according to an embodiment of the present invention.
[FIG. 2] FIG. 2 is a side view according to an embodiment of the present invention.
[FIG. 3] FIG. 3 is an unfolded view and perspective view of a tubular unit body.
[FIG. 4] FIG. 4 is an enlarged perspective view showing a state in which a connecting filament is anchored to a bent portion.
[FIG. 5] FIG. 5 is an unfolded view and perspective view of a tubular unit body.
[FIG. 6] FIG. 6 is an unfolded view and perspective view of a tubular unit body.
[FIG. 7] FIG. 7 is an explanatory diagram showing a connected state by the connecting filament.
[FIG. 8] FIG. 8 is an explanatory diagram showing a connected state by the connecting filament.
[FIG. 9] FIG. 9 is a side view showing a stent in a curved state.
[FIG. 10] FIG. 10 is a perspective view of the stent covered with a tubular cover.
[FIG. 11] FIG. 11 is a perspective view of the stent to which shape-retaining members are attached.
[FIG. 12] FIG. 12 is a plan view showing a mounting state of the shape-retaining members.
[FIG. 13] FIG. 13 is an enlarged perspective view showing a mounting state of the shape-retaining member at a bent portion.
[FIG. 14] FIG. 14 is a perspective view of a stent covered with a tubular cover.
[FIG. 15] FIG. 15 is an explanatory diagram showing a state in which the stent is inserted and positioned in an aorta. Reference Numerals

- 1:: stent
- F:: connecting filament
- U1:: tubular unit body
- U2:: tubular unit body
- U3:: tubular unit body
- V:: tubular unit body
- W:: tubular unit body
- X1:: shape-retaining member
- X2:: shape-retaining member

## Claims

1. A stent formed into a tubular form comprising a plurality of tubular unit bodies arranged in an axial direction, the plurality of tubular unit bodies each being composed of a wire formed into a closed loop and bent in a zigzag manner in a peripheral direction to form a plurality of bent portions spaced apart from one another on both sides, the bent portions each having an anchoring portion, the anchoring portions of the adjacent tubular unit bodies, facing each other, being connected by a connecting filament,
wherein at least some of the tubular unit bodies are connected to the adjacent tubular unit bodies only at the anchoring portions of some of the bent portions of a bent portion group on at least one side so as to be rockably retained.

2. The stent according to claim 1,
wherein the bent portions connected to retain rockably are projected in the axial direction compared to the other bent portions.

3. The stent according to claim 1 or 2, further comprising an elastic shape-retaining member that is attached to the plurality of tubular unit bodies including the rockably retained tubular unit bodies and allows the tubular unit bodies to rock to maintain them in a curved state.

4. The stent according to claim 3,
wherein the shape-retaining member is formed of a curved elastic wire having both ends fixed to the tubular unit bodies, the shape-retaining member being retained by the connecting filament stretched in the peripheral direction so as to conform to the external shape of the stent in the axial direction.

5. The stent according to claim 4,
wherein a pair of the shape-retaining members are fixed at one end to the tubular unit body so as to be spaced apart from each other, crossed over each other, and fixed at the other end to the tubular unit body so as to be spaced apart from each other.

6. The stent according to any one of claims 3 to 5,
wherein the shape-retaining member is composed of a shape memory alloy material.

7. A tubular organ treatment device comprising:
the stent according to any one of claims 1 to 6; and
a tubular cover that covers an inner peripheral surface and/or outer peripheral surface of the stent.
